# EUROPÄISCHE PATENTSCHRIFT

(11) **EP 1 210 060 B1**
(45) Veröffentlichungstag und Bekanntmachung des Hinweises auf die Patenterteilung: **15.09.2004**
(21) Anmeldenummer: 00940315.5
(22) Anmeldetag: 06.06.2000
(51) Int. Cl.: A61K 7/13

(54) **ENTWICKLER-KUPPLER-KOMBINATIONEN**
DEVELOPER-COUPLER COMBINATIONS
COMBINAISONS DEVELOPPEUR-COUPLEUR

(30) Priorität: 15.06.1999 DE 19927074
(43) Veröffentlichungstag der Anmeldung: 05.06.2002
(73) Patentinhaber: Henkel Kommanditgesellschaft auf Aktien, 40589 Düsseldorf (DE)
(72) Erfinder: NAUMANN, Frank, D-40593 Düsseldorf (DE); ROSE, David, D-40723 Hilden (DE); HÖFFKES, Horst, D-40595 Düsseldorf (DE); MEINIGKE, Bernd, D-51381 Leverkusen (DE)
(86) Internationale Anmeldenummer: PCT/EP2000/005155
(87) Internationale Veröffentlichungsnummer: WO 2001/000142

(56) Entgegenhaltungen:
- EP-A- 0 008 079
- DE-A- 1 492 175

## Beschreibung

Diese Erfindung betrifft Färbemittel die als Entwicklerkomponente spezielle Pyridinderivate in Kombination mit bestimmten Kupplerkomponenten enthalten, sowie deren Verwendung zum Färben von Keratinfasern.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kupplerkomponenten die eigentlichen Farbstoffe aus.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Als Kupplerkomponenten werden in der Regel m-Phenylendiaminderivate, Naphthole, Resorcin und Resorcinderivate, Pyrazolone und m-Aminophenole verwendet.

Das europäische Patent dokument EP 0 008 079 A offenbart substituierte Bis-Aminopyridine als Beispiel üblicher Oxidationsfärbemittel mit Entsichtersubstauren (z.B. p-Toluylendiamin) und einer Vupplersubstaur wie z.B. 1-Naphthol oder Resorcin.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkombinationen. Die durch die Farbstoffkombinationen erzielbaren Färbungen sollen beständig sein gegen Licht, Wärme, Schweiß, Reibung und den Einfluß chemischer Reduktionsmittel, z.B. Dauerwellenflüssigkeiten. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Aufgabe der vorliegenden Erfindung war es daher, neue Entwickler-Kupplerkombinationen zu entwickeln, die brillante Färbeergebnisse im Braun-Rot-Bereich ermöglichen.

Es wurde nun überraschenderweise gefunden, daß spezielle Derivate des 2,5-Diaminopyridins in Kombination mit einigen speziellen Kupplerkomponenten zu äußerst kräftigen und intensiven Färbungen mit guten Echtheitseigenschaften führen.

Ein erster Gegenstand der vorliegenden Erfindung sind daher Mittel zum Färben von Keratinfasern, insbesondere menschlichen Haaren, die in einem zum Färben geeigneten Medium
(a) mindestens ein Pyridinderivat der allgemeinen Formel (I) als Entsichterkomponente 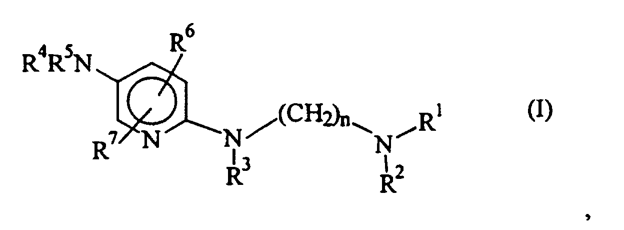 worin R¹ und R² unabhängig voneinander für Wasserstoff, eine C₁- bis C₄-Alkylgruppe oder eine C₁- bis C₄-Hydroxyalkylgruppe stehen oder gemeinsam mit dem Stickstoffatom, an das sie gebunden sind, einen gegebenenfalls substituierten, gesättigten heterocyclischen 5- oder 6-gliedrigen Ring bilden, der ein weiteres Stickstoffatom oder ein Sauerstoffatom enthalten kann, R³ für Wasserstoff, eine C₁- bis C₄-Alkylgruppe oder eine C₁- bis C₄-Hydroxyalkylgruppe steht, R⁴ und R⁵ unabhängig voneinander für Wasserstoff, eine C₁- bis C₄-Alkylgruppe oder eine C₃- bis C₈-Cycloalkylgruppe stehen, R⁶ und R⁷ unabhängig voneinander stehen für Wasserstoff, ein Halogenatom, eine C₁- bis C₄-Alkylgruppe, eine C₁- bis C₄-Hydroxyalkylgruppe, eine C₁- bis C₄-Aminoalkylgruppe oder eine C₁- bis C₄-Alkoxygruppe und n eine ganze Zahl von 1 bis 8 ist, oder eines der physiologisch verträglichen Salze dieser Verbindungen und
(b) mindestens eine Kupplerkomponente, ausgewählt aus einer Gruppe, die gebildet wird von 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2,4-Diaminophenoxyethanol, 2-Chlorresorcin, 4-Chlorresorcin, 2-Chlor-6-methyl-3-aminophenol, 2-Amino-3-hydroxypyridin, 2-Methylresorcin, 5-Methylresorcin, 3-Amino-6-methoxy-2-methylaminopyridin, 2,5-Dimethylresorcin, 2,6-Dihydroxy-3,4-dimethylpyridin, 6-Methyl-1,2,3,4-tetrahydrochinoxalin, o-Aminophenol und 2-Methyl-4-chlor-5-aminophenol,
enthalten.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Besonders bevorzugt im Sinne der vorliegenden Erfindung sind Oxidationsfärbemittel in einem wäßrigen Träger oder in Pulverform.

Beispiele für die als Substituenten in den erfindungsgemäßen Verbindungen der Formel (I) genannten, C₁-bis C₄-Alkylgruppen sind die Gruppen Methyl, Ethyl, Propyl, Isopropyl und Butyl. Ethyl und Methyl sind bevorzugte Alkylgruppen. Eine erfindungsgemäß bevorzugte C₁- bis C₄-Alkoxygruppe, ist beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyloder eine 4-Hydroxybutylgruppe genannt werden. Beispiele für ein Halogenatom sind erfindungsgemäß ein F-, ein Cl- oder ein Br-Atom, ein Cl-Atom ist ganz besonders bevorzugt. Beispiele für die von R¹ und R² gemeinsam mit dem Stickstoffatom gebildeten, gesättigten heterocyclischen 5- oder 6-gliedrigen Ringe sind Morpholin, Piperidin und Pyrrolidin.

Besonders bevorzugt sind Verbindungen, bei denen die Substituenten R³, R⁴, R⁵, R⁶ und R⁷ für Wasserstoff stehen.

Weiterhin bevorzugt sind Verbindungen der Formel (I), bei denen n gleich 2 oder 3 ist.

Ganz besonders bevorzugte Verbindungen der Formel (I) sind 5-Amino-2-(2'aminoethyl)amino-pyridin, 5-Amino-2-(2'-morpholinoethyl)amino-pyridin und 5-Amino-2-(3'-dimethylaminopropyl)amino-pyridin.

Die Herstellung der erfindungsgemäß eingesetzten Entwicklerkomponenten ist bereits in der Literatur beschrieben worden (Takamoto et al., J. Med. Chem. 1994, 37, 18-25).

Besonders bevorzugte Kupplerkomponenten sind 1-Naphthol, 1,5-, 2,7- und 1,7-Dihydroxynaphthalin, 3-Aminophenol, 5-Amino-2-methylphenol, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 2-Amino-3-hydroxypyridin, 3-Amino-6-methoxy-2-methylaminopyridin, 1,3-Bis-(2,4-diaminophenoxy)-propan und 2,4-Diaminophenoxyethanol.

Aus der deutschen Offenlegungsschrift DE-14 92 175 ist bereits der Einsatz bestimmter 2,5-Diamino-pyridinderivate als Entwicklerkomponenten bekannt. Dieser Schrift ist aber keinerlei Hinweis zu entnehmen, daß die erfindungsgemäße Kombination einiger Derivate mit bestimmten Kupplerkomponenten zu derart kräftigen Färbungen im Braun-Rot-Bereich führen.

Sofern es sich bei den erfindungsgemäß verwendeten Substanzen um AminoVerbindungen handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden Pyridinderivate gemäß Formel (I) als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate.

Die erfindungsgemäßen Oxidationsfärbemittel enthalten mindestens eine Entwicklerkomponente gemäß Formel (I) sowie gegebenenfalls eine oder mehrere weitere Entwicklerkomponenten und können gewünschtenfalls noch weitere Kupplerkomponenten enthalten.

Erfindungsgemäß bevorzugte weitere Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxyethyl)-p-phenylendiamin, 2-(2,5-Diaminophenoxy)-ethanol, 1-Phe-nyl-3-carboxyamido-4-amino-pyrazolon-5, 4-Amino-3-methylphenol, 2,4,5,6-Tetraamino-pyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxy-ethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis(N(2-hydroxy-ethyl)-N(4-aminophenylamino))-2-propanol, 4-Amino-2-(2-hydroxyethoxy)-phenol sowie 4,5-Diaminopyrazol-Derivate nach EP 0 740 931 bzw. WO 94/08970 wie z.B. 4,5-Di-amino-1-(2'-hydroxyethyl)-pyrazol.

Besonders bevorzugte weitere Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin, 4-Hydroxy-2,5,6-tri-aminopynmidin, 4,5-Diamino-1-(2-hydroxyethyl)-pyrazol, 2-Aminomethyl-4-amino-phenol, N,N-Bis-(2-hydroxyethyl)-p-phenylendiamin, Bis-(2-hydroxy-5-aminophenyl)-methan und o-Aminophenol.

Erfindungsgemäß bevorzugte weitere Kupplerkomponenten sind:
- m-Aminophenol-Derivate wie beispielsweise 5-(3-Hydroxypropylamino)-2-methylphenol, 2-Hydroxy-4-aminophenoxyethanol, 3-Amino-6-methoxy-2-methylaminophenol, 2,6-Dimethyl-3-aminophenol, 3-Trifluoroacetylamino-2-chlor-6-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol und 3-(Ethylamino)-4-methylphenol,
- o-Aminophenol-Derivate,
- m-Diaminobenzol-Derivate wie 1-Methoxy-2-amino-4-(2'hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Pyrogallol und 1,2,4-Trihydroxybenzol,
- Pyridinderivate wie beispielsweise 2,6-Dihydroxypyridin, 2-Amino-5-chlor-3-hydroxypyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 2-Methyl-1-naphthol, 2-Hydroxymethyl-1-naphthol, 2-Hydroxyethyl-1-naphthol, 1,6-Dihydroxynaphthalin, 1,8-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate,
- Pyrazolderivate,
- Indolderivate wie beispielsweise 4-Hydroxyindol, 6-Hydroxyindol und 7-Hydroxyindol,
- Methylendioxybenzolderivate wie beispielsweise 3,4-Methylendioxyphenol, 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß bevorzugte weitere Kupplerkomponenten sind ferner 3-Dimethylaminophenol, 2,4-Dihydroxyanilin, 8-Amino-6-methoxy-chinolin, 2-Amino-5-naphthol-1,7-disulfonsäure, 3-Amino-1-phenyl-5-pyrazolon, 3,5-Diaminobenzamid, 3-Methylsulfonylamino-2-methylanilin, 5,6-Dihydroxybenzimidazol, 2,2'-Dihydroxybenzylamin, 3,5,3',5'-Tebraamino-2,2'-dimethoxy-diphenyl, 3,5-Diamino-pchlorbenzotrifluorid, 4-Methyl-3-aminophenol, 2,4-Diamino-3-chlorphenol, 1-Amino-3-di-(2-hydroxyethylamino)-4-ethoxybenzol, 2,4-Dimethylresorcin, Bis-(2,4-diaminophenoxy)-methan, 2,6-Bis-(hydroxyethyl)-pyridin, 4-Hydroxy-3-methoxybenzylalkohol, 8-Hydroxychinolin, 4-Hydroxy-3-methoxy-benzylamin, 4-Ethylresorcin, 2-Methylthio-5-aminophenol, 5-[(3-Hydroxypropyl)amino]-2-methylphenol, 2,6-Dimethoxy-3-aminophenol und 2,6-Diamino-3-methylthiotoluol.

Diese weiteren Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Färbemittel können die Oxidationsfarbstoffvorprodukte in einen geeigneten wasserhaltigen Träger eingearbeitet werden. Solche Träger sind zum Zwecke der Haarfärbung z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO^{(⁻} ⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropion-säuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsar-cosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammo-niumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkyl-ammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere, quaternierter Polyvinylalkohol und Polyquaternium-2
- zwitterionische und amphotere Polymere wie beispielsweise Acrylamidopropyl-trimethylammoniumchlorid/Acrylat-Copolymere und Octylacrylamid/Methylmethacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein zweiter Gegenstand der vorliegenden Erfindung ist die Verwendung der vorgenannten Mittel zum Färben keratinischer Fasern.

Die nachfolgenden Beispiele sollen den Erfindungsgegenstand näher erläutern.

### Ausführungsbeispiele

### 1.1 Herstellung der Färbecreme

| Teilmischung A | |
|---|---|
| Hydrenol® D¹ | 8,50g |
| Lorol® techn.² | 2,00g |
| Eumulgin® B2³ | 0,75g |
| Texapon® NSO⁴ | 20,00g |
| Dehyton® K⁵ | 12,50g |
| Wasser | 30,00g |

| | |
|---|---|
| ¹ C₁₆₋₁₈-Fettalkohol (INCI-Bezeichnung: Cetearyl alcohol) (HENKEL) | |
| ² C₁₂₋₁₈-Fettalkohol (INCI-Bezeichnung: Coconut alcohol) (HENKEL) | |
| ³ Cetylstearylalkohol mit ca. 20 EO-Einheiten (INCI-Bezeichnung: Ceteareth-20) (HENKEL) | |
| ⁴ Laurylethersulfat, Natriumsalz (ca. 27,5% Aktivsubstanz; INCI-Bezeichnung: Sodium Laureth Sulfate) (HENKEL) | |
| ⁵ N,N-Dimethyl-N-(C₈₋₁₈-kokosamidopropyl)ammoniumacetobetain (ca. 30% Aktivsubstanz; INCI-Bezeichnung: Aqua (Water), Cocamidopropyl Betaine) (HENKEL) | |

Die Substanzen Hydrenol® D, Lorol® und Eumulgin® B2 wurden bei 80°C aufgeschmolzen, mit dem 80°C heißem Wasser, enthaltend Texapon® NSO und Dehyton® K, vermischt und unter starkem Rühren emulgiert. Danach wurde die Emulsion unter schwachem Rühren abgekühlt.

| Teilmischung B | |
|---|---|
| Natriumsulfit | 1,00g |
| Ammoniumsulfat | 1,00g |
| Farbstoffvorprodukte | jeweils 2,5mmol |
| Ammoniak (25%ige Lösung) | ad pH=10,0 |
| Wasser | 10,00g |

Die Farbstoffvorprodukte wurden in dem 50°C heißem Wasser unter Zugabe von Natriumsulfit, Ammoniumsulfat und Ammoniak gelöst.

Die Farbstoffvorproduktlösung (Teilmischung B) wurde zur Emulsion (Teilmischung A) gegeben, mit Ammoniak auf pH=10 eingestellt und mit Wasser auf 100 g aufgefüllt. Es wurde bis zum Erreichen der Raumtemperatur weitergerührt.

### 1.2 Färbung der keratinischen Fasern

Die so erhaltene Färbecreme wurde im Verhältnis 1:1 mit einer 1%-igen H₂O₂-Lösung vermischt und auf 5cm lange Strähnen standardisierten, zu 80% ergrauten, aber nicht besonders vorbehandelten Menschenhaares (Kerling) aufgetragen. Nach 30min Einwirkzeit bei 32°C wurde das Haar gespült, mit einem üblichen Haarwaschmittel ausgewaschen und anschließend getrocknet.

Die Ergebnisse der Färbeversuche sind den folgenden Tabellen zu entnehmen.

**Tabelle I:**

| Ausfärbungen | | |
|---|---|---|
| Entwickler | Kuppler | Nuance der gefärbten Strähne |
| 5-Amino-2-(2'-aminoethyl)amino-pyridin | Resorcin | Kuba |
| 5-Amino-2-(2'-aminoethyl)amino-pyridin | 3-Amino-6-methoxy-2-methylaminopyridin | Dunkelbraun |
| 5-Amino-2-(2'-aminoethyl)amino-pyridin | 5-Amino-2-methylphenol | Rotbraun |
| 5-Amino-2-(2'-aminoethyl)amino-pyridin | 2,4-Diaminophenoxyethanol | Dunkelbraun |
| 5-Amino-2-(2'-aminoethyl)amino-pyridin | 1,3-Bis-(2,4-diaminophenoxy)-propan-tetrahydrochlorid | Dunkelbraun |
| 5-Amino-2-(2'-aminoethyl)amino-pyridin | 4-Chlorresorcin | Rotbraun |
| 5-Amino-2-(3'-dimethylaminopropyl)-amino-pyridin | Resorcin | Photobraun |
| 5-Amino-2-(3'-dimethylaminopropyl)-amino-pyridin | 3-Amino-6-methoxy-2-methylaminopyridin | Negerbraun |
| 5-Amino-2-(3'-dimethylaminopropyl)-amino-pyridin | 5-Amino-2-methylphenol | Somali |
| 5-Amino-2-(3'-dimethylaminopropyl)-amino-pyridin | 2,4-Diaminophenoxyethanol | Rehbraun |
| 5-Amino-2-(3'-dimethylaminopropyl)-amino-pyridin | 1,3-Bis-(2,4-diaminophenoxy)-propan-tetrahydrochlorid | Dunkelbraun |
| 5-Amino-2-(3'-dimethylaminopropyl)-amino-pyridin | 4-Chlorresorcin | Rotbraun |
| 5-Amino-2-(2'-morpholino-ethyl)amino-pyridin | Resorcin | Mattrot |
| 5-Amino-2-(2'-morpholino-ethyl)amino-pyridin | 3-Amino-6-methoxy-2-methylaminopyridin | Olivbraun |
| 5-Amino-2-(2'-morpholino-ethyl)amino-pyridin | 5-Amino-2-methylphenol | Braunorange |
| 5-Amino-2-(2'-morpholino-ethyl)amino-pyridin | 2,4-Diaminophenoxyethanol | Haarbraun |
| 5-Amino-2-(2'-morpholino-ethyl)amino-pyridin | 1,3-Bis-(2,4-diaminophenoxy)-propan-tetrahydrochlorid | Schokoladenbraun |
| 5-Amino-2-(2'-morpholino-ethyl)amino-pyridin | 4-Chlorresorcin | Rotbraun |

## Patentansprüche

1. Oxidationsfärbemittel zum Färben von Keratinfasern, insbesondere menschlichen Haaren, **dadurch gekennzeichnet, daß** es in einem zum Färben geeigneten Medium
(a) mindestens ein Pyridinderivat der allgemeinen Formel (I) als Entwichterkomponente 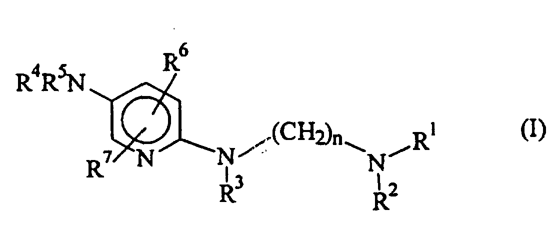 worin R¹ und R² unabhängig voneinander für Wasserstoff, eine C₁- bis C₄-Alkylgruppe oder eine C₁- bis C₄-Hydroxyalkylgruppe stehen oder gemeinsam mit dem Stickstoffatom, an das sie gebunden sind, einen gegebenenfalls substituierten, gesättigten heterocyclischen 5- oder 6-gliedrigen Ring bilden, der ein weiteres Stickstoffatom oder ein Sauerstoffatom enthalten kann, R³ für Wasserstoff, eine C₁- bis C₄-Alkylgruppe oder eine C₁- bis C₄-Hydroxyalkylgruppe steht, R⁴ und R⁵ unabhängig voneinander für Wasserstoff, eine C₁- bis C₄-Alkylgruppe oder eine C₃- bis C₈-Cycloalkylgruppe stehen, R⁶ und R⁷ unabhängig voneinander stehen für Wasserstoff, ein Halogenatom, eine C₁- bis C₄-Alkylgruppe, eine C₁- bis C₄-Hydroxyalkylgruppe, eine C₁- bis C₄-Aminoalkylgruppe oder eine C₁- bis C₄-Alkoxygruppe und n eine ganze Zahl von 1 bis 8 ist, oder eines der physiologisch verträglichen Salze dieser Verbindungen und
(b) mindestens eine Kupplerkomponente, ausgewählt aus einer Gruppe, die gebildet wird von 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2,4-Diaminophenoxyethanol, 2-Chlorresorcin, 4-Chlorresorcin, 2-Chlor-6-methyl-3-aminophenol, 2-Amino-3-hydroxypyridin, 2-Methylresorcin, 5-Methylresorcin, 3-Amino-6-methoxy-2-methylaminopyridin, 2,5-Dimethylresorcin, 2,6-Dihydroxy-3,4-dimethylpyridin, 6-Methyl-1,2,3,4-tetrahydrochinoxalin, o-Aminophenol und 2-Methyl-4-chlor-5-aminophenol, enthält.

2. Mittel nach Anspruch 1, **dadurch gekennzeichnet, daß** die Reste R³, R⁴, R⁵, R⁶ und R⁷ der Verbindung der Formel (I) für Wasserstoff stehen.

3. Mittel nach einem der Ansprüche 1 oder 2, **dadurch gekennzeichnet, daß** die Verbindung der Formel (I) 5-Amino-2-(2'-aminoethyl)amino-pyridin ist.

4. Mittel nach einem der Ansprüche 1 oder 2, **dadurch gekennzeichnet, daß** die Verbindung der Formel (I) 5-Amino-2-(3'-dimethylaminopropyl)amino-pyridin ist.

5. Mittel nach einem der Ansprüche 1 oder 2, **dadurch gekennzeichnet, daß** die Verbindung der Formel (I) 5-Amino-2-(2'-morpholinoethyl)amino-pyridin ist.

6. Mittel nach einem der Ansprüche 1 bis 5, **dadurch gekennzeichnet, daß** die Kupplerkomponente (b) ausgewählt ist aus einer Gruppe, die gebildet wird von 1-Naphthol, 1,5-, 2,7- und 1,7-Dihydroxynaphthalin, 3-Aminophenol, 5-Amino-2-methylphenol, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-diaminopyridin, 2-Amino-3-hydroxypyridin, 3-Amino-6-methoxy-2-methylaminopyridin, 1,3-Bis-(2,4-diaminophenoxy)-propan und 2,4-Diaminophenoxyethanol.

7. Mittel nach einem der Ansprüche 1 bis 6, **dadurch gekennzeichnet, daß** es mindestens eine weitere Entwicklerkomponente, ausgewählt aus der Gruppe, die aus p-Phenylendiamin, p-Toluylendiamin, p-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxyethyl)-pyrazol, 2-Aminomethyl-4-amino-phenol, N,N-Bis-(2-hydroxyethyl)-p-phenylendiamin, Bis-(2-hydroxy-5-aminophenyl)-methan und o-Aminophenol gebildet wird, enthält.

8. Mittel nach einem der Ansprüche 1 bis 7, **dadurch gekennzeichnet, daß** Entwicklerkomponenten in einer Menge von 0,005 bis 20 Gew.-%, vorzugsweise 0,1 bis 5 Gew.-%, und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

9. Mittel nach einem der Ansprüche 1 bis 8, **dadurch gekennzeichnet, daß** mindestens ein direktziehender Farbstoff enthalten ist.

10. Verwendung von Mitteln nach einem der Ansprüche 1 bis 9 zum Färben keratinischer Fasern.

## Claims

1. An oxidation colorant for colouring keratin fibres, more particularly human hair, **characterized in that** it contains in a medium suitable for colouring
(a) at least one pyridine derivative corresponding to general formula (I): 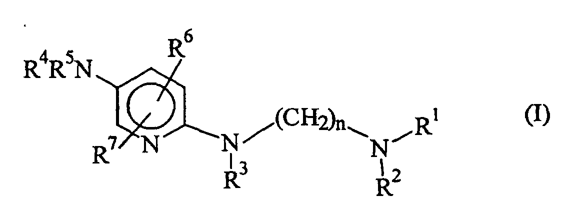 in which R¹ and R² independently of one another represent hydrogen, a C₁₋₄ alkyl group or a C₁₋₄ hydroxyalkyl group or, together with the nitrogen atom to which they are attached, an optionally substituted, saturated heterocyclic 5- or 6-membered ring which may contain another nitrogen atom or an oxygen atom, R³ represents hydrogen, a C₁₋₄ alkyl group or a C₁₋₄ hydroxyalkyl group, R⁴ and R⁵ independently of one another represent hydrogen, a C₁₋₄ alkyl group or a C₃₋₈ cycloalkyl group, R⁶ and R⁷ independently of one another represent hydrogen, a halogen atom, C₁₋₄ alkyl group, a C₁₋₄ hydroxyalkyl group, a C₁₋₄ aminoalkyl group or a C₁₋₄ alkoxy group and n is an integer of 1 to 8,
or a physiologically compatible salt of these compounds as primary intermediate and
(b) at least one secondary intermediate selected from the group consisting of 1-naphthol, 1,5-, 2,7- and 1,7-dihydroxynaphthalene, 5-amino-2-methylphenol, m-aminophenol, resorcinol, resorcinol monomethylether, m-phenylenediamine, 1-phenyl-3-methyl-5-pyrazolone, 2,4-dichloro-3-aminophenol, 1,3-bis-(2,4-diaminophenoxy)-propane, 2,4-diaminophenoxyethanol, 2-chlororesorcinol, 4-chlororesorcinol, 2-chloro-6-methyl-3-aminophenol, 2-amino-3-hydroxypyridine, 2-methyl resorcinol, 5-methyl resorcinol, 3-amino-6-methoxy-2-methylaminopyridine, 2,5-dimethyl resorcinol, 2,6-dihydroxy-3,4-dimethylpyridine, 6-methyl-1,2,3,4-tetrahydroquinoxaline, o-aminophenol and 2-methyl-4-chloro-5-aminophenol.

2. A colorant as claimed in claim 1, **characterized in that** the substituents R³, R⁴, R⁵, R⁶ and R⁷ of the compound of formula (I) represent hydrogen.

3. A colorant as claimed in claim 1 or 2, **characterized in that** the compound of formula (I) is 5-amino-2-(2'-aminoethyl)-aminopyridine.

4. A colorant as claimed in claim 1 or 2, **characterized in that** the compound of formula (I) is 5-amino-2-(3'-dimethylaminopropyl)-aminopyridine.

5. A colorant as claimed in claim 1 or 2, **characterized in that** the compound of formula (I) is 5-amino-2-(2'-morpholinoethyl)-aminopyridine.

6. A colorant as claimed in any of claims 1 to 5, **characterized in that** the secondary intermediate (b) is selected from the group consisting of 1-naphthol, 1,5-, 2,7- and 1,7-dihydroxynaphthalene, 3-aminophenol 5-amino-2-methylphenol, resorcinol, 4-chlororesorcinol, 2-chloro-6-methyl-3-aminophenol, 2-methyl resorcinol, 5-methyl resorcinol, 2,5-dimethylresorcinol, 2,6-dihydroxy-3,4-diaminopyridine, 2-amino-3-hydroxypyridine, 3-amino-6-methoxy-2-methylaminopyridine, 1,3-bis-(2,4-diaminophenoxy)-propane and 2,4-diaminophenoxyethanol.

7. A colorant as claimed in any of claims 1 to 6, **characterized in that** it contains at least one another primary intermediate selected from the group consisting of p-phenylenediamine, p-toluylenediamine, p-aminophenol, 1-(2'-hydroxyethyl)-2,5-diaminobenzene, 4-amino-3-methylphenol, 2,4,5,6-tetra-aminopyrimidine, 2-hydroxy-4,5,6-triaminopyrimidine, 4-hydroxy-2,5,6-triaminopyrimidine, 4,5-diamino-1-(2-hydroxyethyl)-pyrazole, 2-aminomethyl-4-aminophenol, N,N-bis-(2-hydroxyethyl)-p-phenylenediamine, bis-(2-hydroxy-5-aminophenyl)-methane and o-aminophenol.

8. A colorant as claimed in any of claims 1 to 7, **characterized in that** primary intermediates are present in a quantity of 0.005 to 20% by weight and preferably 0.1 to 5% by weight and secondary intermediates are present in a quantity of 0.005 to 20% by weight and preferably 0.1 to 5% by weight, based on the oxidation colorant as a whole.

9. A colorant as claimed in any of claims 1 to 8, **characterized in that** it contains at least one substantive dye.

10. The use of the colorant claimed in any of claims 1 to 9 for colouring keratinous fibres.

## Revendications

1. Agent colorant par oxydation pour teindre des fibres de kératine, en particulier des cheveux humains, **caractérisé en ce qu'**il contient dans un milieu convenant à la teinture
(a) au moins un dérivé pyridine de formule générale (I) en tant que composant révélateur, 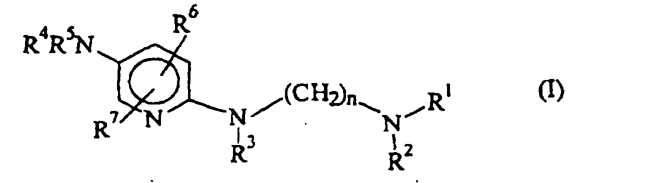 dans laquelle R¹ et R² représentent indépendamment l'un de l'autre un atome d'hydrogène, un groupe alkyle en C₁-C₄ ou un groupe hydroxyalkyle en C₁ à C₄ ou bien conjointement avec l'atome d'azote auquel ils sont liés, ils forment un cycle de 5 ou 6 maillons, hétérocyclique, saturé, éventuellement substitué, qui peut contenir un atome d'azote supplémentaire ou un atome d'oxygène, R³ représente un atome d'hydrogène, un groupe alkyle en C₁-C₄ ou un groupe hydroxyalkyle en C₁ à C₄, R⁴ et R⁵ représentent indépendamment l'un de l'autre, un atome d'hydrogène, un groupe alkyle en C₁ à C₄ ou un groupe cycloalkyle en C₃ à C₈, R⁶ et R⁷ représentent indépendamment l'un de l'autre, un atome d'hydrogène, un atome d'halogène, un groupe alkyle en C₁ à C₄, un groupe hydroxyalkyle en C₁ à C₄, un groupe aminoalkyle en C₁ à C₄ ou un groupe alcoxy en C₁ à C₄ et n est un nombre entier de 1 à 8, ou un des sels acceptables sur le plan physiologique de ces composés, et
(b) au moins un composant coupleur, choisi dans un groupe qui est formé par le 1-naphtol, le 1,5-, 2,7- et 1,7-dihydroxynaphtalène, le 5-amino-2-méthylphénol, le m-aminophénol, la résorcine, l'éther monométhylique de résorcine, la m-phénylènediamine, le 1-phényl-3-méthyl-pyrazolone-5, le 2,4-dichloro-3-aminophénol, le 1,3-bis-(2,4-diaminophénoxy)-propane, le 2,4-diaminophénoxyéthanol, la 2-chlororésorcine, la 4-chlororésorcine, le 2-chloro-6-méthyl-3-aminophénol, la 2-amino-3-hydroxypyridine, la 2-méthylrésorcine, la 5-méthylrésorcine, la 3-amino-6-méthoxy-2-méthyl-aminopyridine, la 2,5-diméthylrésorcine, la 2,6-dihydroxy-3,4-diméthyl-pyridine, la 6-méthyl-1,2,3,4-tétrahydroquinoxaline, le o-aminophénol, et le 2-méthyl-4-chloro-5-aminophénol.

2. Agent selon la revendication 1, **caractérisé en ce que** les résidus R³, R⁴, R⁵, R⁶ et R⁷ du composé de formule (I) représentent un atome d'hydrogène.

3. Agent selon l'une quelconque des revendications 1 ou 2, **caractérisé en ce que** le composé de formule (I) est la 5-amino-2-(2'-amino-éthyl)aminopyridine.

4. Agent selon l'une quelconque des revendications 1 ou 2, **caractérisé en ce que** le composé de formule (I) est la 5-amino-2-(3'-diméthylaminopropyl)amino-pyridine.

5. Agent selon l'une quelconque des revendications 1 ou 2, **caractérisé en ce que** le composé de formule (I) est la 5-amino-2-(2'-morpholinoéthyl)aminopyridine.

6. Agent selon l'une quelconque des revendications 1 à 5, **caractérisé en ce que**, le composant coupleur (b) est choisi dans un groupe qui est formé par le 1-naphtol, le 1,5-, 2,7- et 1,7-dihydroxynaphtalène, le 3-aminophénol, le 5-amino-2-méthylphénol, la résorcine, la 4-chlororésorcine, le 2-chloro-6-méthyl-3-aminophénol, la 2-méthylrésorcine, la 5-méthylrésorcine, la 2,5-diméthylrésorcine, la 2,6-dihydroxy-3,4-diaminopyridine, la 2-amino-3-hydroxypyridine, la 3-amino-6-méthoxy-2-méthylaminopyridine, le 1,3-bis(2,4-diaminophénoxy)propane et le 2,4-diaminophénoxyéthanol.

7. Agent selon l'une quelconque des revendications 1 à 6, **caractérisé en ce qu'**il contient au moins un composant révélateur supplémentaire, choisi dans le groupe qui est formé par la p-phénylènediamine, la p-toluylènediamine, le p-aminophénol, le 1-(2'-hydroxyéthyl)-2,5-diaminobenzène, le 4-amino-3-méthylphénol, la 2,4,5,6-tétraaminopyrimidine, la 2-hydroxy-4,5,6-triaminopyrimidine, la 4-hydroxy-2,5,6-triaminopyrimidine, le 4,5-diamino-1-(2-hydroxyéthyl)-pyrazole, le 2-aminométhyl-4-amino-phénol, la N,N-bis-(2-hydroxyéthyl)-p-phénylènediamine, le bis-(2-hydroxy-5-amino-phényl)méthane et le o-aminophénol.

8. Agent selon l'une quelconque des revendications 1 à 7, **caractérisé en ce que** des composants révélateurs sont contenus en une quantité de 0,005 à 20% en poids, de préférence de 0,1 à 5% en poids, et des composants coupleurs en une quantité de 0,005 à 20% en poids, de préférence de 0,1 à 5% en poids, respectivement par rapport à tout l'agent colorant par oxydation.

9. Agent selon l'une quelconque des revendications 1 à 8, **caractérisé en ce qu'**au moins un colorant montant directement sur les fibres y est contenu.

10. Utilisation d'agents selon l'une quelconque des revendications 1 à 9, pour teindre des fibres de kératine.
